(19) Europäisches Patentamt
European Patent Office
Office européen des brevets

(11) Veröffentlichungsnummer: **0 196 547**
**A1**

(12) **EUROPÄISCHE PATENTANMELDUNG**

(21) Anmeldenummer: 86103686.1

(22) Anmeldetag: 18.03.86

(51) Int. Cl.4: C 07 D 277/82
C 07 D 263/58, A 01 N 43/76
A 01 N 43/78

(30) Priorität: 30.03.85 JP 65018/85

(43) Veröffentlichungstag der Anmeldung:
08.10.86 Patentblatt 86/41

(84) Benannte Vertragsstaaten:
AT BE CH DE FR GB IT LI NL SE

(71) Anmelder: NIHON TOKUSHU NOYAKU SEIZO K.K.
No.4, 2-chome, Nihonbashi Honcho Chuo-ku
Tokyo 103(JP)

(72) Erfinder: Kume, Toyohiko
6-7-8, Asahigaoka
Hino-shi Tokyo(JP)

(72) Erfinder: Tsuboi, Shinichi
3-26-1, Hirayama
Hino-shi Tokyo(JP)

(72) Erfinder: Isono, Kunihiro
2-32-4, Asahigaoka
Hino-shi Tokyo(JP)

(72) Erfinder: Sasaki, Shoko
1-7-3-1341 Higashi-Hirayama
Hino-shi Tokyo(JP)

(72) Erfinder: Hattori, Yumi
598 Kobiki-Cho Hachioji-Shi
Tokyo(JP)

(74) Vertreter: Ernst, Hilmar, Dr.
Bayer AG Konzernverwaltung RP Patentabteilung
D-5090 Leverkusen, Bayerwerk(DE)

(54) **Neue Benzamide.**

(57) Offenbart werden neue Benzamide der Formel (I)

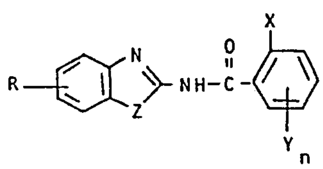

(I)

und die Verwendung der neuen Verbindungen als Insektizide.

EP 0 196 547 A1

NIHON TOKUSHU NOYAKU SEIZO K.K.
Tokyo / Japan



0196547

# Neue Benzamide

Die vorliegende Erfindung betrifft neue Benzamide, ein Verfahren zu ihrer Herstellung sowie ihre Verwendung als Pestizid, insbesondere als Insektizid.

Es wurde bereits offenbart, daß bestimmte Benzamide, z.B. N-(6-Chlorobenzothiazol-2-yl)-2,6-difluorobenzamid, insektizide Aktivitäten besitzen (siehe J. Agric. Food Chem. 24 (1976), Seiten 1065-1068).

Nunmehr wurden neue Benzamide der Formel (I)

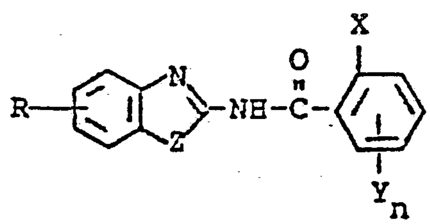

(I)

gefunden, in der

X ein Halogen-Atom, eine Niederalkyl-Gruppe, eine Niederalkoxy-Gruppe oder eine Halogen-substituierte Niederalkyl-Gruppe bezeichnet,

Y ein Halogen-Atom oder eine Niederalkyl-Gruppe bezeichnet,

n 0, 1 oder 2 bezeichnet,

Z ein Sauerstoff- oder Schwefel-Atom bezeichnet und

R eine Halogen-substituierte Niederalkyl-Gruppe, eine Halogen-substituierte Niederalkoxy-Gruppe, eine Halogen-substituierte Niederalkylthio-Gruppe, eine Halogen-substituierte Niederalkylsulfinyl-Gruppe oder eine Halogen-substituierte Niederalkylsulfonyl-Gruppe bezeichnet.

Die Verbindungen der Formel (I) werden erhalten, wenn die Verbindungen der Formel (II)

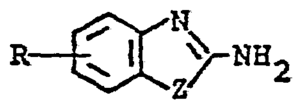

(II)

,

in der Z und R die angegebenen Bedeutungen haben, mit Verbindungen der Formel (III)

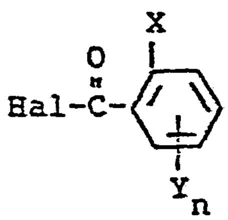

(III)

,

in der X, Y und n die angegebenen Bedeutungen haben und Hal ein Halogen-Atom ist, umgesetzt werden, gegebenenfalls in Gegenwart inerter Lösungsmittel und in Gegenwart säurebindender Mittel.

Die neuen Benzamide zeigen hervorragende insektizide Wirkung.

Überraschenderweise zeigen die Benzamide gemäß der vorliegenden Erfindung eine im wesentlichen stark überlegene insektizide Wirksamkeit, speziell gegen Larven

von Insekten der Ordnung Lepidoptera, im Vergleich zu analogen Verbindungen, die aus dem oben zitierten Stand der Technik bekannt sind.

Die Niederalkyl-Gruppen X und Y sind geradkettig oder verzweigt und enthalten 1 bis 8, vorzugsweise 1 bis 6 und besonders bevorzugt 1 bis 4 Kohlenstoff-Atome. Beispiele, die erwähnt werden können, sind Methyl, Ethyl, n- und i-Propyl sowie n-, i-, sec- und tert-Butyl. Bevorzugt werden Methyl und Ethyl, und besonders bevorzugt wird Methyl.

Die Niederalkoxy-Gruppen X sind geradkettig oder verzweigt und enthalten 1 bis 8, vorzugsweise 1 bis 6 und besonders bevorzugt 1 bis 4 Kohlenstoff-Atome. Beispiele, die erwähnt werden können, sind Methoxy, Ethoxy, n- und i-Propoxy sowie n-, i-, sec- und tert-Butoxy. Bevorzugt werden Methoxy und Ethoxy, und besonders bevorzugt wird Methoxy.

Die Halogen-substituierten Niederalkyl-Gruppen X sind geradkettig oder verzweigt und enthalten 1 bis 8, vorzugsweise 1 bis 6 und besonders bevorzugt 1 bis 4 Kohlenstoff-Atome und 1 bis 7, vorzugsweise 1 bis 5 und besonders bevorzugt 1 bis 3 (gleichartige oder unterschiedliche) Halogen-Atome. Halogen-Atome sind Fluor, Chlor, Brom und Iod, vorzugsweise Fluor, Chlor und Brom und besonders bevorzugt Fluor und Chlor. Beispiele, die erwähnt werden können, sind $CHF_2$, $CF_3$, $CF_2CF_3$, $CH_2CF_3$ und $CF_2CHCl_2$, wobei die $CF_3$-Gruppe besonders bevorzugt ist.

Die Niederalkyl-Gruppen in den Halogen-substituierten Niederalkyl-, Niederalkoxy-, Niederalkylthio-, Niederalkylsulfinyl- oder Niederalkylsulfonyl-Gruppen R sind geradkettig oder verzweigt und enthalten 1 bis 8, vorzugsweise 1 bis 4 und besonders bevorzugt 1 oder 2 Kohlenstoff-Atome. Beispiele, die erwähnt werden können, sind Methyl, Ethyl, n- und i-Propyl sowie n-, i-, sec- und tert-Butyl. Sie können durch 1 bis 7, vorzugsweise 1 bis 5 und besonders bevorzugt 1 bis 3 (gleichartige oder unterschiedliche) Halogen-Atome substituiert sein. Bevorzugte Halogen-Atome sind Fluor, Chlor, Brom und Iod. Besonders bevorzugt werden Fluor und Chlor. Beispiele, die erwähnt werden können, sind $CF_3$, $CF_2CHCl_2$, $OCF_3$, $OCHF_2$, $OCF_2CHF_2$, $OCF_2CF_3$, $OCH_2CF_3$, $OCF_2CHCl_2$, $OCF_2$, $CHFCF_3$, $SCHF_2$, $SCF_3$, $SCF_2CH_2F$, $SCF_2CF_3$, $SOCF_3$, $SOCH_2CF_3$, $SO_2CF_3$ und $SO_2CH_2CF_3$. Besonders bevorzugt werden $CF_3$, $OCF_3$ und $SCF_3$. Vorzugsweise steht R in der 4-, 5- oder 6-Stellung, besonders bevorzugt in der 6-Stellung, des Benzothiazol-2-yl- oder Benzoxazol-2-yl-Restes.

In den allgemeinen Formeln (I) und (III) bezeichnen X und Y vorzugsweise Halogen.

Die Halogene X und Y in den Formeln (I) und (III) bedeuten Fluor, Chlor, Brom oder Iod, vorzugsweise Fluor, Chlor und Brom und besonders bevorzugt Fluor und Chlor.

In den allgemeinen Formeln (I) und (II) bezeichnet Z vorzugsweise Schwefel.

n in den allgemeinen Formeln (I) und (III) steht vorzugsweise für 0 oder 1.

In dem Fall, in dem n 2 bedeutet, befinden sich die Substituenten Y vorzugsweise in 2- und 4-Stellung oder

in 4- und 6-Stellung des Benzoyl-Restes, und in dem Fall, in dem n 1 bedeutet, befindet sich der Substituent Y vorzugsweise in der 4-, 5- oder 6-Stellung, besonders bevorzugt in der 6-Stellung des Benzoyl-Restes.

Bevorzugte Verbindungen der Formel (I) sind diejenigen, in denen

X Halogen, $C_1$-$C_4$-Alkyl, $C_1$-$C_4$-Alkoxy oder Halogeno-$C_1$-$C_4$-alkyl (vorzugsweise Halogen) bezeichnet,

Y Halogen oder $C_1$-$C_4$-Alkyl (vorzugsweise Halogen) bezeichnet,

n 0, 1 oder 2 (vorzugsweise 0 oder 1) bezeichnet,

Z Sauerstoff oder Schwefel (vorzugsweise Schwefel) bezeichnet und

R einen durch Halogen (vorzugsweise Fluor und/oder Chlor) substituierten Rest aus der Gruppe $C_1$-$C_4$-Alkyl, $C_1$-$C_4$-Alkoxy, $C_1$-$C_4$-Alkylthio, $C_1$-$C_4$-Alkylsulfinyl und $C_1$-$C_4$-Alkylsulfonyl (vorzugsweise $C_1$-$C_4$-Alkyl, $C_1$-$C_4$-Alkoxy und $C_1$-$C_4$-Alkylthio) bezeichnet.

Bevorzugte Verbindungen der Formel (I) sind diejenigen, in denen X Chlor, Fluor, Methyl, Methoxy oder Trifluoromethyl bezeichnet, Y Chlor, Fluor oder Methyl bezeichnet, n 0, 1 oder 2 bezeichnet, Z ein Schwefel-Atom bezeichnet und R eine Fluor-substituierte Alkyl-Gruppe mit 1 oder 2 Kohlenstoff-Atomen, eine Fluor-substituierte Alkoxy-Gruppe mit 1 oder 2 Kohlenstoff-Atomen oder eine Fluor-substituierte Alkylthio-Gruppe mit 1 oder 2 Kohlenstoff-Atomen bezeichnet.

Von den Verbindungen der Formel (I) mit den oben angebenen bevorzugten Definitionen sind diejenigen

besonders bevorzugt, in denen X Chlor oder Fluor ist, Y Fluor ist, n 0 oder 1 ist, Z ein Schwefel-Atom ist und R eine Trifluoromethyl- oder Trifluoromethoxy-Gruppe ist.

Darüber hinaus sind zusätzlich zu den vorstehenden Definitionen der bevorzugten oder besonders bevorzugten Verbindungen die am meisten bevorzugten Verbindungen der Formel (I) diejenigen, in denen R in der 6-Stellung des Benzothiazols substituiert ist.

Die folgenden Verbindungen seien als spezielle Beispiele für die Verbindungen der Formel (I) erwähnt:
N-(6-Trifluoromethylbenzothiazol-2-yl)-2,6-difluorobenzamid und
N-(6-Trifluoromethoxybenzothiazol-2-yl)-2,6-difluorobenzamid,

Wenn in dem vorstehenden Verfahren 2-Amino-6-trifluoromethylbenzothiazol und 2,6-Difluorobenzoylchlorid als Ausgangsstoffe eingesetzt werden, kann die Reaktion durch das folgende Schema veranschaulicht werden:

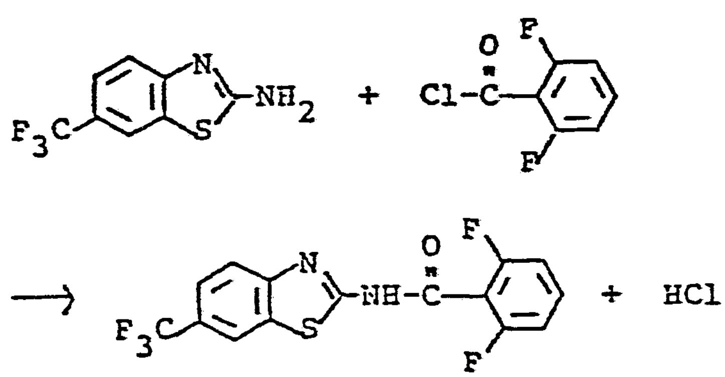

Die Verbindungen der Formel (II) sind bekannte Verbindungen (siehe z.B. die US-PS 2 832 761, Zh. Obshch. Khim. 33 (7), 2301-7 (1963) und die EP-Patentveröffentlichung 0 050 551). Zu speziellen Beispielen zählen
2-Amino-6-trifluoromethylbenzothiazol,
2-Amino-6-trifluoromethoxybenzothiazol,
2-Amino-6-difluoromethoxybenzothiazol,
2-Amino-6-(1,1,2,2-tetrafluoroethoxy)benzothiazol und
2-Amino-6-trifluoromethylthiobenzothiazol.

Die im Vorstehenden beispielhaft genannten Verbindungen der Formel (II) können aus substituierten Anilinen oder substituierten o-Hydroxyanilinen nach an sich bekannten Methoden hergestellt werden, die beispielsweise in J. Chem. Soc. 1969, 268, in der JP-OS 59 679/1984, in der DE-OS 26 01 700, in J. Pharm. Soc., Japan, 73, 1312 (1953) und in J. Am. Chem. Soc. 71, 3417, beschrieben sind.

Die Verbindungen der Formel (III) sind bekannt. Zu speziellen Beispielen zählen
2-Chlorobenzoylchlorid,
2-Chloro-6-fluorobenzoylchlorid,
2,6-Difluorobenzoylchlorid,
2-Fluorobenzoylchlorid,
2-Methylbenzoylchlorid,
2-Ethylbenzoylchlorid,
2-Methoxybenzoylchlorid,
2,6-Dichlorobenzoylchlorid,
2-Trifluoromethylbenzoylchlorid,
2,4,6-Trifluorobenzoylchlorid sowie
die entsprechenden Bromide.

Das vorstehende Verfahren kann nach dem bekannten, in der US-PS 3 555 157 beschriebenen Verfahren durchgeführt werden.

Bei der Durchführung der vorgenannten Verfahren können sämtliche inerten Lösungsmittel als geeignete Verdünnungsmittel dienen.

Zu Beispielen für solche Verdünnungsmittel zählen aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Diisopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Ester wie Ethylacetat und Amylacetat; Säureamide wie Dimethylformamid und Dimethylacetamid; Sulfone und Sulfoxide wie Dimethylsulfoxid und Sulfolan; und Basen wie Pyridin oder 4-Dimethylaminopyridin.

Das vorstehende Verfahren kann in Gegenwart eines säurebindenden Mittels durchgeführt werden. Zu Beispielen für derartige säurebindenden Mittel zählen organische tertiäre Amine wie Triethylamin, Dimethylanilin, Pyridin und Lutidin sowie anorganische Basen, beispielsweise Alkalimetallhydroxide und -carbonate wie Natriumhydroxid, Kaliumhydroxid, Natriumcarbonat und Kaliumcarbonat.

Das vorstehende Verfahren kann in einem weiten Temperaturbereich durchgeführt werden, beispielsweise bei einer Temperatur zwischen etwa -20°C und dem Siedepunkt der Mischung, vorzugsweise zwischen etwa 0°C und etwa 100°C. Die Reaktion wird zweckmäßigerweise unter normalem Atmosphärendruck durchgeführt, jedoch ist es auch möglich, unter erhöhtem oder vermindertem Druck zu arbeiten.

In der Praxis des vorstehenden Verfahrens können die neuen Verbindungen der Formel (I) dadurch erhalten werden, daß vorzugsweise 1 mol der Verbindung der Formel (II) mit etwa 0,8 bis 1,2 mol, besonders bevorzugt 1 bis etwa 1,1 mol, der Verbindung der Formel (III) in einem inerten Lösungsmittel in Gegenwart eines säurebindenden Mittels umgesetzt wird.

Die aktiven Verbindungen der Formel (I) gemäß der vorliegenden Erfindung zeigen starke pestizide, insbesondere arthropodizide und besonders bevorzugt insektizide Aktivität und können aus diesem Grunde besonders bevorzugt als Insektizide eingesetzt werden. Die aktiven Verbindungen (I) der vorliegenden Erfindung zeigen einen genauen Bekämpfungseffekt gegen Schadinsekten, ohne Schäden bei Kulturpflanzen zu verursachen. Weiterhin können die Verbindungen der vorliegenden Erfindung zur Bekämpfung und Ausrottung eines weiten Spektrums von Schädlingen verwendet werden, darunter saugende Insekten, beißende Insekten und andere Pflanzenparasiten, Schädlingen in Getreidevorräten und gelagerten Körnerfrüchten und gesundheitsgefährdenden Schädlingen.

Beispiele für die Schädlinge sind im Folgenden angegeben.

Insekten der Ordnung Coleoptera

Callosobruchus chinensis,
Sitophilus zeamais,
Tribolium castaneum,
Epilachna vigintioctomaculata,
Agriotes fuscicollis,
Anomala rufocuprea,
Leptinotarsa decemlineata,
Diabrotica spp.,
Monochamus alternatus,
Lissorhoptus oryzophilus und
Lyctus brunneus;

Insekten der Ordnung Lepidoptera

Lymantria dispar,
Malacosoma neustria,
Pieris rapae,
Spodoptera litura,
Mamestra brassicae,
Chilo suppressalis,
Pyrausta nubilalis,
Ephestia cautella,
Adoxophyes orana,
Carpocapsa pomonella,
Agrotis fucosa,
Galleria mellonella,
Heliotis virescens,
Plutella maculipennis und
Phyllocnistis citrella;

Insekten der Ordnung Diptera

Musca domestica,
Aedes aegypti,

Hylemia platura,
Culex pipiens,
Anopheles sinensis und
Culex tritaeniorhynchus.

Auf dem Gebiet der Tierhaltung und Tierzucht sind die neuen Verbindungen gemäß der vorliegenden Erfindung gegenüber verschiedenen schädlichen Tierparasiten wirksam. Beispiele für solche Tierparasiten sind Gastrophilus spp. und Stomoxys spp.

Die aktiven Verbindungen können in gebräuchliche Formulierungen überführt werden, etwa als Lösungen, Emulsionen, Suspensionen, Pulver, Schäume, Pasten, Granulat, Aerosol, mit der aktiven Verbindung imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist auch möglich, farbgebende Mittel, etwa anorganische Pigmente wie beispielsweise Eisenoxid, Titanoxid und Preußisch Blau oder organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% der aktiven Verbindung.

Die aktiven Verbindungen gemäß der Erfindung können in ihren handelsüblichen Formulierungen oder den aus diesen Formulierungen hergestellten Anwendungsformen im Gemisch mit anderen bekannten aktiven Verbindungen vorliegen, etwa mit Insektiziden, Ködern, Sterilisationsmitteln, Akariziden, Nematiziden, Fungiziden, Wachstumsregulatoren oder Herbiziden. Zu den Insektiziden

gehören beispielsweise Phosphate, Carbamate, Carboxylate, chlorierte Kohlenwasserstoffe, Phenylharnstoffe und von Mikroorganismen erzeugte Substanzen.

Die aktiven Verbindungen gemäß der vorliegenden Erfindung können weiterhin in ihren handelsüblichen Formulierungen oder den aus diesen Formulierungen hergestellten Anwendungsformen im Gemisch mit synergistischen Mitteln vorliegen. Synergistische Mittel sind Verbindungen, die die Wirkung der aktiven Verbindungen steigern, ohne daß es für das zugesetzte synergistische Mittel erforderlich ist, selbst aktiv zu sein.

Der Gehalt der aktiven Verbindung in den aus den im Handel erhältlichen Formulierungen hergestellten Anwendungsformen kann innerhalb weiter Grenzen variieren. Die Konzentration der aktiven Verbindung in den Anwendungsformen kann 0,0000001 bis 100 Gew.-% betragen und liegt vorzugsweise zwischen 0,0001 und 1 Gew.-%.

Die Verbindungen werden in üblicher Weise in einer den Anwendungsformen angemessenen Form zur Anwendung gebracht.

Bei der Verwendung gegen Hygiene-Schädlinge und Schädlinge in Produkt-Vorräten zeichnen sich die aktiven Verbindungen durch eine hervorragende Rückstandswirkung auf Holz und Ton sowie eine gute Beständigkeit gegen Alkali auf gekalkten Unterlagen aus.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

Herstellungsbeispiele

Beispiel 1

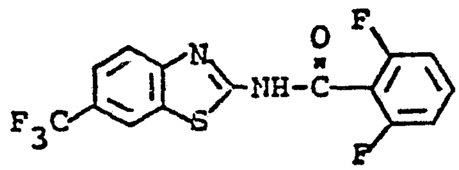

(Verbindung Nr. 1)

Bei 0°C bis 5°C wurde 2,6-Difluorobenzoylchlorid (1,77 g) tropfenweise zu einer Lösung aus 2-Amino-6-trifluoromethylbenzothiazol (2,18 g), Tetrahydrofuran (30 ml) und Triethylamin (1,1 g) hinzugefügt. Die Reaktionsmischung wurde 5 h bei 30°C bis 40°C gerührt, und dann wurde das Tetrahydrofuran abgedampft. Der feste Rückstand wurde mit Wasser gewaschen und aus Ethanol umkristallisiert, wonach das gewünschte N-(6-Trifluoromethylbenzothiazol-2-yl)-2,6-difluorobenzamid (2,8 g) erhalten wurde. Schmp. 256-257°C.

Beispiel 2

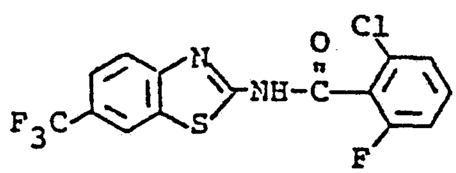

(Verbindung Nr. 2)

Bei 0°C bis 5°C wurde 2-Chloro-6-fluorobenzoylchlorid (1,93 g) tropfenweise zu einer Lösung aus 2-Amino-6-trifluoromethylbenzothiazol (2,18 g), Pyridin (30 ml) und 4-Dimethylaminopyridin (0,1 g) hinzugefügt. Die

Reaktionsmischung wurde 1 d bei Raumtemperatur (20°C bis 30°C) gerührt, und dann wurde das Pyridin abgedampft. Dem Rückstand wurde Wasser zugesetzt, und die Mischung wurde gerührt. Die erhaltenen Kristalle wurden durch Filtration gesammelt und gründlich mit Wasser gewaschen. Umkristallisation aus Ethanol lieferte das gewünschte N-(6-Trifluoromethylbenzothiazol-2-yl)-2-chloro-6-fluorobenzamid (2,5 g). Schmp. 265-270°C.

Die nachstehende Tabelle 1 zeigt neue Benzamide der allgemeinen Formel (I) gemäß der vorliegenden Erfindung, die nach den gleichen Verfahrensweisen wie in den obigen Beispielen hergestellt wurden.

In Tabelle 1 bedeutet die Bezeichnung " - " in der " $Y_n$ " überschriebenen Spalte die Abwesenheit eines Substituenten.

Tabelle 1

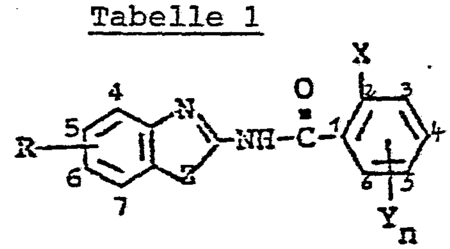

| Verbindung Nr. | R | Z | X | $Y_n$ | Physikal. Konstante |
|---|---|---|---|---|---|
| 3 | 6-$CF_3$ | S | Cl | - | Schmp. 225-226°C |
| 4 | 4-$CF_3$ | S | F | 6-F | Schmp. 215-216°C |
| 5 | 5-$CF_3$ | S | F | 6-F | Schmp. 214-216°C |
| 6 | 6-$CF_3$ | O | F | 6-F | |
| 7 | 6-$OCF_3$ | S | Cl | - | Schmp. 195-197°C |
| 8 | 6-$CCF_3$ | S | F | 6-F | Schmp. 215-216°C |
| 9 | 6-$SCF_3$ | S | Cl | - | Schmp. 206-207°C |

Nit 192

Tabelle 1 - Fortsetzung

| Verbindung Nr. | R | Z | X | $Y_n$ | Physikal. Konstante |
|---|---|---|---|---|---|
| 10 | $6-SCF_3$ | S | F | 6-F | Schmp. 210-211°C |
| 11 | $6-\overset{O}{S}-CF_3$ | S | F | 6-F | |
| 12 | $6-\overset{O}{\underset{O}{S}}-CF_3$ | S | F | 6-F | Schmp. 208-210°C |
| 13 | $6-SCH_2CF_3$ | S | F | 6-F | Schmp. 160-161°C |
| 14 | $6-\overset{O}{\underset{O}{S}}CH_2CF_3$ | S | F | 6-F | |
| 15 | $6-OCHF_2$ | S | F | 6-F | Schmp. 196.5-197.5°C |
| 16 | $6-OCF_2CHF_2$ | S | F | 6-F | Schmp. 210-212°C |
| 17 | $6-OCF_2CF_3$ | S | F | 6-F | |
| 18 | $6-OCF_2CHCl_2$ | S | F | 6-F | |
| 19 | $6-OCF_2CHFCF_3$ | S | F | 6-F | Schmp. 186-187°C |
| 20 | $6-CF_3$ | S | $-CH_3$ | - | Schmp. 200-201°C |
| 21 | $6-CF_3$ | S | $-C_2H_5$ | - | |
| 22 | $6-CF_3$ | S | F | - | Schmp. 198-199°C |
| 23 | $6-OCF_3$ | S | Cl | 4-Cl | Schmp. 211-211.5°C |
| 24 | $6-CF_3$ | S | F | $4,6-F_2$ | |
| 25 | $6-OCF_3$ | S | F | $6-CH_3$ | |
| 26 | $6-OCF_3$ | S | F | $4,6-F_2$ | |

Nit 192

0196547

Tabelle 1 - Fortsetzung

| Verbindung Nr. | R | Z | X | $Y_n$ | Physikal. Konstante |
|---|---|---|---|---|---|
| 27 | $6\text{-}OCF_3$ | S | Cl | 6-F | |
| 28 | $6\text{-}OCF_3$ | O | Cl | 6-F | |
| 29 | $6\text{-}OCF_3$ | S | $-OCH_3$ | - | Schmp. 211-212°C |
| 30 | $6\text{-}OCF_3$ | S | Br | - | Schmp. 208-209°C |
| 31 | $6\text{-}OCF_3$ | S | $-CF_3$ | - | Schmp. 164-165°C |
| 32 | $6\text{-}CF_3$ | S | F | 6-Cl | Schmp. 265-270°C |
| 33 | $6\text{-}OCF_2CHF_2CF_3$ | S | Cl | - | Schmp. 167-169°C |

Verwendungsbeispiele

Vergleichs-Verbindung A:

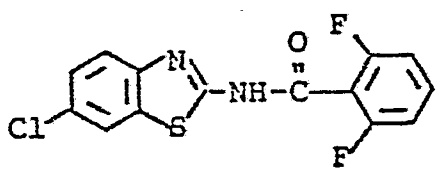

(die in J. Agric. Food Chem. 24 (1976), Seiten 1065-1068 beschriebene Verbindung)

Beispiel 3

Test mit Larven von Spodoptera litura:

Herstellung einer Test-Chemikalie:

| | | |
|---|---|---|
| Lösungsmittel: | 3 Gew.-Teile | Dimethylformamid |
| Emulgator: | 1 Gew.-Teil | Polyoxyethylenalkylphenylether |

Zur Herstellung eines geeigneten Wirkstoff-Präparats wurde 1 Gew.-Teil der aktiven Verbindung mit dem Lösungsmittel und dem Emulgator in den oben angegebenen Mengen vermischt. Die Mischung wurde mit Wasser auf eine vorher festgesetzte Konzentration verdünnt.

Test-Verfahren:

Kohlblätter wurden in die wäßrige Verdünnung der aktiven Verbindung mit der vorbestimmten Konzentration getaucht. Nach dem Luft-Trocknen der Chemikalie wurden die Kohlblätter in Petrischalen von 9 cm Durchmesser gelegt. 5 Larven im 3. Stadium von Spodoptera litura wurden in der Petrischale ausgesetzt, und die Schalen wurden in einen thermostatisierten Raum von 28°C gestellt. 7 d danach wurde das Vernichtungsverhältnis berechnet.

Die mit typischen Verbindungen erhaltenen Ergebnisse sind in Tabelle 2 aufgeführt.

Tabelle 2

| Verbindung Nr. | Wirkstoff-Konzentration ppm | Vernichtungs-verhältnis % |
|---|---|---|
| 1 | 2 | 100 |
| 2 | 10 | 100 |
| 3 | 10 | 100 |
| 8 | 2 | 100 |
| 10 | 10 | 100 |
| Vergleichs-Verbindung A | 40 | 80 |
| | 10 | 0 |

## Beispiel 4

### Test mit Larven von Plutella maculipennis:

Test-Verfahren:
Kohlblätter wurden in die wäßrige Verdünnung der aktiven Verbindung mit der vorbestimmten Konzentration getaucht, die wie in Beispiel 3 hergestellt wurde. Nach dem Luft-Trocknen der Chemikalie wurden die Kohlblätter in Petrischalen von 9 cm Durchmesser gelegt. 10 Larven von Plutella maculipennis wurden in der Petrischale ausgesetzt, und die Schalen wurden in einen thermostatisierten Raum von 23°C gestellt. 7 d danach wurde das Vernichtungsverhältnis berechnet.
Die mit typischen Verbindungen erhaltenen Ergebnisse sind in Tabelle 3 aufgeführt.

Tabelle 3

| Verbindung Nr. | Wirkstoff-Konzentration ppm | Vernichtungsverhältnis % |
|---|---|---|
| 1. | 10 | 100 |
| 2 | 40 | 100 |
| 3 | 40 | 100 |
| 7 | 10 | 100 |
| 8 | 2 | 100 |
| Vergleichs-Verbindung A | 200 | 100 |
| | 40 | 0 |
| | 2 | 0 |

## P a t e n t a n s p r ü c h e

1. Benzamid der Formel (I)

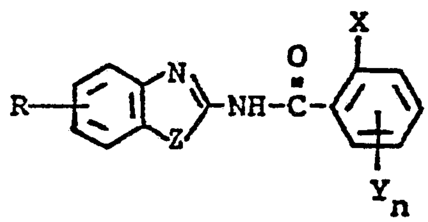

(I)

,

in der

X ein Halogen-Atom, eine Niederalkyl-Gruppe, eine Niederalkoxy-Gruppe oder eine Halogen-substituierte Niederalkyl-Gruppe bezeichnet,

Y ein Halogen-Atom oder eine Niederalkyl-Gruppe bezeichnet,

n 0, 1 oder 2 bezeichnet,

Z ein Sauerstoff- oder Schwefel-Atom bezeichnet und

R eine Halogen-substituierte Niederalkyl-Gruppe, eine Halogen-substituierte Niederalkoxy-Gruppe, eine Halogen-substituierte Niederalkylthio-Gruppe, eine Halogen-substituierte Niederalkylsulfinyl-Gruppe oder eine Halogen-substituierte Niederalkylsulfonyl-Gruppe bezeichnet.

2. Verbindungen der Formel (I) nach Anspruch 1, dadurch gekennzeichnet, daß

X Halogen, $C_1$-$C_4$-Alkyl, $C_1$-$C_4$-Alkoxy oder Halogeno-$C_1$-$C_4$-alkyl (vorzugsweise Halogen) bezeichnet,

Y Halogen oder $C_1$-$C_4$-Alkyl (vorzugsweise Halogen) bezeichnet,

n 0, 1 oder 2 (vorzugsweise 0 oder 1) bezeichnet,

Z Sauerstoff oder Schwefel (vorzugsweise Schwefel) bezeichnet und

R einen durch Halogen (vorzugsweise Fluor und/oder Chlor) substituierten Rest aus der Gruppe $C_1$-$C_4$-Alkyl, $C_1$-$C_4$-Alkoxy, $C_1$-$C_4$-Alkylthio, $C_1$-$C_4$-Alkylsulfinyl und $C_1$-$C_4$-Alkylsulfonyl (vorzugsweise $C_1$-$C_4$-Alkyl, $C_1$-$C_4$-Alkoxy und $C_1$-$C_4$-Alkylthio) bezeichnet.

3. Verbindungen der Formel (I) nach Anspruch 1, dadurch gekennzeichnet, daß
X Chlor, Fluor, Methyl, Methoxy oder Trifluoromethyl bezeichnet, Y Chlor, Fluor oder Methyl bezeichnet, n 0, 1 oder 2 bezeichnet, Z ein Schwefel-Atom bezeichnet und R eine Fluor-substituierte Alkyl-Gruppe mit 1 oder 2 Kohlenstoff-Atomen, eine Fluor-substituierte Alkoxy-Gruppe mit 1 oder 2 Kohlenstoff-Atomen oder eine Fluor-substituierte Alkylthio-Gruppe mit 1 oder 2 Kohlenstoff-Atomen bezeichnet.

4. Verbindungen der Formel (I) nach Anspruch 1, dadurch gekennzeichnet, daß
X Chlor oder Fluor ist, Y Fluor ist, n 0 oder 1 ist, Z ein Schwefel-Atom ist und R eine Trifluoromethyl- oder Trifluoromethoxy-Gruppe ist.

5. Verbindungen nach Ansprüchen 1 bis 4, ausgewählt aus den Verbindungen der Formeln

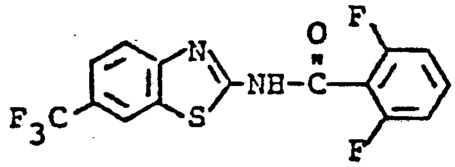

und

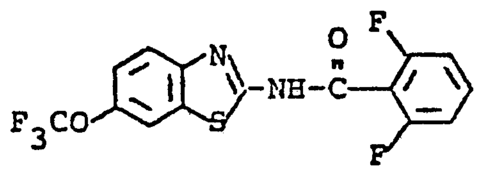

.

6. Verfahren zur Herstellung neuer Benzamide der Formel (I)

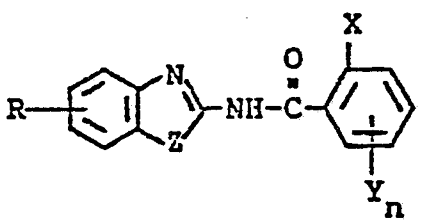

(I)

,

in der

X ein Halogen-Atom, eine Niederalkyl-Gruppe, eine Niederalkoxy-Gruppe oder eine Halogen-substituierte Niederalkyl-Gruppe bezeichnet,

Y ein Halogen-Atom oder eine Niederalkyl-Gruppe bezeichnet,

n 0, 1 oder 2 bezeichnet,

Z ein Sauerstoff- oder Schwefel-Atom bezeichnet und

R eine Halogen-substituierte Niederalkyl-Gruppe, eine Halogen-substituierte Niederalkoxy-Gruppe,

eine Halogen-substituierte Niederalkylthio-Gruppe, eine Halogen-substituierte Niederalkylsulfinyl-Gruppe oder eine Halogen-substituierte Niederalkylsulfonyl-Gruppe bezeichnet,

dadurch gekennzeichnet, daß die Verbindungen der Formel (II)

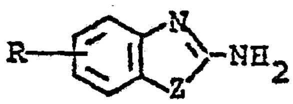

(II)

,

in der Z und R die angegebenen Bedeutungen haben, mit Verbindungen der Formel (III)

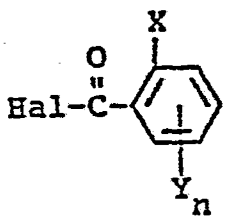

(III)

,

in der X, Y und n die angegebenen Bedeutungen haben und Hal ein Halogen-Atom ist, umgesetzt werden, gegebenenfalls in Gegenwart inerter Lösungsmittel und in Gegenwart säurebindender Mittel.

7. Pestizide, vorzugsweise insektizide, Mittel, dadurch gekennzeichnet, daß sie wenigstens eines der Benzamide der Formel (I) nach Ansprüchen 1 bis 6 enthalten.

8. Verfahren zur Bekämpfung von Schädlingen, insbesondere von Insekten, dadurch gekennzeichnet, daß man Benzamide der Formel (I) nach Ansprüchen 1 bis 6 auf Schädlinge und/oder deren Lebensraum einwirken läßt.

9. Verwendung von Benzamiden der Formel (I) nach Ansprüchen 1 bis 6 zur Bekämpfung von Schädlingen, vorzugsweise von Insekten.

10. Verfahren zur Herstellung von pestiziden, insbesondere insektiziden, Mitteln, dadurch gekennzeichnet, daß Benzamide der Formel (I) nach Ansprüchen 1 bis 6 mit Streckmitteln und/oder grenzflächenaktiven Mittel vermischt werden.

Europäisches Patentamt

0196547

# EUROPÄISCHER RECHERCHENBERICHT

Nummer der Anmeldung

EP 86103686.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| A | DE - A1 - 2 813 826 (NIHON TOKUSHU NOYAKU SEIZO K.K.)<br>* Ansprüche 1-6 *<br>-- | 1,6-10 | C 07 D 277/82<br>C 07 D 263/58<br>A 01 N 43/76<br>A 01 N 43/78 |
| D,A | US - A - 3 555 157 (RUBERT F. LINDE-MANN)<br>* Zusammenfassung *<br>-- | 1,6-10 | |
| A | EP - A1 - 0 062 254 (NIHOÑ TUKUSHU NOYAKU SEIZO K.K.)<br>* Ansprüche 1,2 *<br>-- | 1,6 | |
| A | Chemical Abstracts, Band 93, Nr. 3, 21. Juli 1980, Columbus, Ohio, USA<br>AUGUSTIN, M.; RICHTER, M.; SALAS, S. "Reactions with N-acylimino dithiocarbonic acid diesters" Seite 693, Spalte 1, Zusammenfassung-Nr. 26 307a<br>& J. Prakt. Chem. 1980, 322(1); 55-68<br>---- | 1,6 | RECHERCHIERTE SACHGEBIETE (Int. Cl.4)<br>C 07 D 277/00<br>C 07 D 263/00 |

Der vorliegende Recherchenbericht wurde für alle Patentansprüche erstellt.

| Recherchenort | Abschlußdatum der Recherche | Prüfer |
|---|---|---|
| WIEN | 27-06-1986 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument